# EUROPEAN PATENT APPLICATION

(11) **EP 3 514 544 A1**
(43) Date of publication of application: **24.07.2019**
(21) Application number: 17851111.9
(22) Date of filing: 13.09.2017
(51) Int. Cl.: G01N 33/566, G01N 33/569, G01N 33/543

(54) **METHOD AND DEVICE FOR DIFFERENTIALLY DIAGNOSING ENCEPHALITIS ON BASIS OF TRAIL**

(30) Priority: 13.09.2016 KR 20160118262
(71) Applicant: Advanced NT, Seoul 03087 (KR); Ant Labs Inc., Seoul 03127 (KR)
(72) Inventor: CHU, Kon, Seoul 05834 (KR); LEE, Sang Kun, Seoul 06600 (KR); JUNG, Keun Hwa, Seoul 04423 (KR); LEE, Soon Tae, Seoul 04322 (KR); KIM, Man Ho, Seoul 06305 (KR); MOON, Jang Sup, Seoul 06705 (KR)
(74) Representative: Viering, Jentschura & Partner mbB Patent- und Rechtsanwälte
(86) International application number: PCT/KR2017/010022
(87) International publication number: WO 2018/052237

(57) **Abstract**

The present invention relates an information providing method comprising the steps of acquiring a biological sample separated from a specimen, identifying concentration of a protein, TNF-related apoptosis-inducing ligand (TRAIL) in the cerebrospinal fluid from the said biological sample, comparing the concentration of the said identified TRAIL to a reference value, and providing information related to encephalitis disorder according to the said results of comparison.

## Description

### Field of the Invention

The present invention relates to a method and an apparatus for providing information on encephalitis related diseases. More specifically, the present invention relates to TRAIL-based encephalitis differentiation diagnosis.

### Description of the Prior Art

Encephalitis is a neurologic disorder that can lead to severe late disorders if accurate diagnosis and appropriate treatment are not performed exactly. Encephalitis can be divided into infectious encephalitis and autoimmune encephalitis.

Infectious encephalitis can be caused by a wide variety of causative strains such as bacteria, viruses, fungi, and the like, and only therapeutic agents for some causative strains of them are present. It takes a long time to diagnose infectious encephalitis, and there is a problem that the diagnosis rate for pathogenic bacteria is only about 50%.

Autoimmune encephalitis is a newly diagnosed disease within the recent about 10 years, which is a 20-30% of total encephalitis patients. In the case of autoimmune encephalitis, autoimmune encephalitis is usually diagnosed by autoantibody detection. However, there are various types of causative strains and new antibodies are continuously discovered. Therefore, it is difficult to diagnose autoimmune encephalitis by conventional antibody test kit alone.

As such, existing diagnostic methods have difficulties in discriminating and accurately diagnosing infectious encephalitis and autoimmune encephalitis. Therefore, there is an acute need for a method and apparatus for providing information related to encephalitis diseases with faster and more accurately.

### SUMMARY OF THE INVENTION

Accordingly, it is an object of the present invention to provide a method and apparatus for providing information on autoimmune encephalitis and viral encephalitis disorder more accurately.

In order to achieve the above object, according to an embodiment of the present invention, it is characterized in that an information providing method according to an embodiment of the present invention comprises the steps of:
acquiring a biological sample separated from a specimen,
identifying concentration of a protein, TNF-related apoptosis-inducing ligand (TRAIL) in the cerebrospinal fluid from the said biological sample,
comparing the concentration of the said identified TRAIL to a reference value, and
providing information related to encephalitis disorder according to the said results of comparison.

Furthermore, the said comparing step may further include a steps of discriminating the possibility of an autoimmune encephalitis disorder if the concentration of the identified TRAIL is less than a reference value according to the result of the comparison; and discriminating the possibility of a viral encephalitis disorder if the concentration of the identified TRAIL is greater than a reference value according to the result of the comparison.

Furthermore, according to another embodiment of the present invention, the said information providing method may further comprise the steps of providing viral encephalitis diagnostic information according to the viral encephalitis disorder possibility discrimination, and providing autoimmune encephalitis diagnostic information according to the autoimmune encephalitis disorder possibility discrimination.

Furthermore, the step of discriminating the possibility of autoimmune encephalitis disorder may comprise a step of discriminating between anti-NMDA receptor encephalitis and LGI1 encephalitis according to the said identified TRAIL concentration.

Furthermore, when the concentration of the TRAIL is lower than a predetermined value, it may further comprise a step of discriminating with normal without encephalitis.

Furthermore, the step of discriminating the possibility of the viral encephalitis disorder may comprise a step of discriminating Japanese Encephalitis virus (JEV) encephalitis and Herpesviridae encephalitis according to the said identified TRAIL concentration.

Furthermore, the concentration of TRAIL in the cerebrospinal fluid may be characterized by being obtained from an enzyme immunoassay (ELISA).

The apparatus for providing information according to an embodiment of the present invention includes a data input unit for receiving data including a concentration of TRAIL (TNF-related apoptosis-inducing ligand), which is a protein in cerebrospinal fluid obtained from a biological sample, a data comparing unit for comparing the said concentration of TRAIL with the reference value, and an information providing unit for providing information related to encephalitis disorder according to the said comparison result.

Furthermore, the said information providing unit may provide information related to an autoimmune encephalitis disorder when the concentration of the TRAIL is lower than a reference value according to the said results of comparison, and provide information related to a viral encephalitis disorder when the concentration of the TRAIL is greater than a reference value according to the said results of comparison.

Furthermore, the said information related to a viral encephalitis disorder may include the diagnostic information according to discrimination of the possibility of a viral encephalitis disorder, and the said information related to an autoimmune encephalitis disorder may include the diagnostic information according to discrimination of the possibility of a autoimmune encephalitis disorder.

Furthermore, it is characterized in that the said information providing unit may distinguish between the anti-NMDA receptor encephalitis and the LGI1 encephalitis according to the said results of comparison.

Furthermore, the said information providing unit can distinguish JEV (Japanese Encephalitis virus) encephalitis and Herpesviridae encephalitis according to the said results of comparison.

Being constituting as the above, the method and apparatus for diagnosing encephalitis according to the present invention has an effect that can discriminate between viral encephalitis and autoimmune encephalitis using a TNF-related apoptosis-inducing ligand (TRAIL) in cerebrospinal fluid so that it possible to provide information related to encephalitis disorder more exactly and rapidly. Thereby, the encephalitis treatment according to the cause can be performed promptly so that it is possible to treat the encephalitis more effectively.

The effects of the present invention described above are merely one of various effects according to the present invention, and the present invention can be realized in various forms according to the application mode of the embodiment.

### BRIEF DESCRIPTION OF THE DRAWINGS

The combustion promoter according to the present invention will now be described by way of example only, with reference to the accompanying drawings, in which:
Figure 1 is a graph analyzing the concentration (pg/ml) of TRAIL in cerebrospinal fluid.
Figure 2 is a bar graph showing the mean values of TRAIL concentration in cerebrospinal fluid of normal subjects, patients with autoimmune encephalitis, and patients with viral encephalitis.
Figure 3 is a bar graph showing mean values of TRAIL concentration in cerebrospinal fluid of normal subjects, patients with NMDA receptor encephalitis, patients with LGI1 encephalitis, patients with Japanese encephalitis (JEV), and patients with Herpesviridae encephalitis.
Figure 4 is the ROC curves showing for discriminating viral encephalitis using TRAIL concentration in cerebrospinal fluid of normal subjects, patients with autoimmune encephalitis, and patients with viral encephalitis.
Figure 5 is the ROC curves showing for discriminating viral encephalitis using TRAIL concentration in cerebrospinal fluid of patients with autoimmune encephalitis, and patients with viral encephalitis.
Figure 6 shows an apparatus capable of providing information related to encephalitis disorder.

### DETAILED DESCRIPTION OF THE INVENTION

Hereinafter, preferred embodiments of the present invention will be described in detail. The foregoing objects, features and advantages of the present invention will become more apparent from the following detailed description taken in conjunction with the accompanying figures. Hereinafter, preferred embodiments of the present invention will be described in detail with reference to the accompanying figures. Like reference numerals designate like elements throughout the specification. Furthermore, in the following description, the well-known functions or constructions are not described in detail to avoid obscuring the subject matter of the present invention.

A method for providing information according to an embodiment of the present invention may comprise the steps of:
acquiring a biological sample separated from a specimen,
identifying concentration of a protein, TNF-related apoptosis-inducing ligand (TRAIL) in the cerebrospinal fluid from the said biological sample,
comparing the concentration of the said identified TRAIL to a reference value, and
providing information related to encephalitis disorder according to the said results of comparison.

Furthermore, the said comparing step may further include a steps of discriminating the possibility of an autoimmune encephalitis disorder if the concentration of the identified TRAIL is less than a reference value according to the result of the comparison; and discriminating the possibility of a viral encephalitis disorder if the concentration of the identified TRAIL is greater than a reference value according to the result of the comparison.

Furthermore, according to another embodiment of the present invention, the said information providing method may further comprise the steps of providing viral encephalitis diagnostic information according to the viral encephalitis disorder possibility discrimination, and providing autoimmune encephalitis diagnostic information according to the autoimmune encephalitis disorder possibility discrimination.

Furthermore, the step of discriminating the possibility of autoimmune encephalitis disorder may comprise a step of discriminating between anti-NMDA receptor encephalitis and LGI1 encephalitis according to the said identified TRAIL concentration.

Furthermore, when the concentration of the TRAIL is lower than a predetermined value, it may further comprise a step of discriminating with normal without encephalitis.

Furthermore, the step of discriminating the possibility of the viral encephalitis disorder may comprise a step of discriminating Japanese Encephalitis virus (JEV) encephalitis and Herpesviridae encephalitis according to the said identified TRAIL concentration.

Furthermore, the concentration of TRAIL in the cerebrospinal fluid may be characterized by being obtained from an enzyme immunoassay (ELISA).

The apparatus for providing information according to an embodiment of the present invention includes a data input unit for receiving data including a concentration of TRAIL (TNF-related apoptosis-inducing ligand), which is a protein in cerebrospinal fluid obtained from a biological sample, a data comparing unit for comparing the said concentration of TRAIL with the reference value, and an information providing unit for providing information related to encephalitis disorder according to the said comparison result.

Furthermore, the said information providing unit may provide information related to an autoimmune encephalitis disorder when the concentration of the TRAIL is lower than a reference value according to the said results of comparison, and provide information related to a viral encephalitis disorder when the concentration of the TRAIL is greater than a reference value according to the said results of comparison.

Furthermore, the said information related to a viral encephalitis disorder may include the diagnostic information according to discrimination of the possibility of a viral encephalitis disorder, and the said information related to an autoimmune encephalitis disorder may include the diagnostic information according to discrimination of the possibility of a autoimmune encephalitis disorder.

Furthermore, it is characterized in that the said information providing unit may distinguish between the anti-NMDA receptor encephalitis and the LGI1 encephalitis according to the said results of comparison.

Furthermore, the said information providing unit can distinguish JEV (Japanese Encephalitis virus) encephalitis and Herpesviridae encephalitis according to the said results of comparison.

For the examples of the present invention, TRAIL enzyme immunoassay (ELISA) was performed using a stored cerebrospinal fluid of 10 control subjects which have not encephalitis, 10 autoimmune encephalitis confirmed patients, and 10 viral encephalitis confirmed patients (Human TRAIL/TNFSF10 quantikine ELISA Kit, R&D System). At this time, all used cerebrospinal fluid (CSF) is initial CSF before suspected encephalitis patients start treatment by visiting.

On the other hand, in 10 patients confirmed with autoimmune encephalitis, 5 patients with anti-NMDA receptor encephalitis and 5 patients with LGI1 encephalitis were enrolled. In 10 patients confirmed with viral encephalitis, 2 patients with Varicella Zoster Virus encephalitis, 2 patients with Herpes Simplex Virus type 2 encephalitis, 1 patient with CytoMegaloVirus encephalitis, and 5 patients with Japanese Encephalitis virus encephalitis were enrolled.

### 1. Analysis of TRAIL concentration in cerebrospinal fluid.

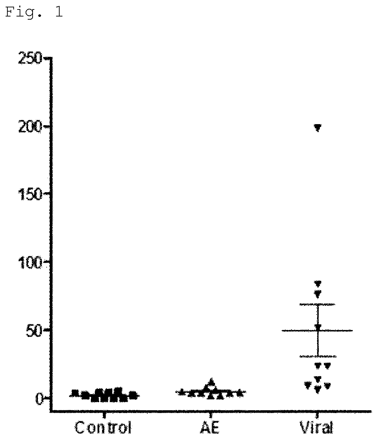

Figure 1 shows graphically the TRAIL concentrations in the cerebrospinal fluid (CSF) of the control subjects group, the patient group with confirmed autoimmune encephalitis, and the patient group with confirmed viral encephalitis by performing the same method as described above substantially. The abscissa of the graph in FIG. 1 indicates the control subjects group (Control), the patient group with confirmed autoimmune encephalitis (AE), and the patient group with confirmed viral encephalitis (Viral), and the vertical axis represents concentration (pg/ml). The values of the 30 patients belonging to the control group and the experimental group were expressed as square in the control group (n = 10), triangle in the autoimmune encephalitis confirmed group (n = 10) and inverted triangle in the viral encephalitis confirmed group (n = 10).

Referring for figure 1, the TRAIL concentration in the cerebrospinal fluid of the control group and the autoimmune encephalitis confirmed group was similar value each other, but the TRAIL concentration in the cerebrospinal fluid of the viral encephalitis confirmed group was higher than that of the control group and the autoimmune encephalitis confirmed group.

### 2. Mean analysis of TRAIL concentration in cerebrospinal fluid between groups.

The Mean, Standard Deviation (SD) and Standard Error of Mean (SEM) for TRAIL concentrations in cerebrospinal fluid of the control group, the autoimmune encephalitis confirmed group, and the viral encephalitis confirmed group were described in Table 1 below.

**Table 1**

| Group | Mean | Standard Deviation (SD) | Standard Error of Mean (SEM) |
|---|---|---|---|
| control subjects group (Control) (n = 10) | 2.04pg/ml | 1.99pg/ml | 0.63pg/ml |
| patient group with autoimmune encephalitis (AE) (n = 10) | 5.08pg/ml | 3.08pg/ml | 0.97pg/ml |
| the patient group with viral encephalitis (Viral) (n = 10) | 49.42pg/ml | 59.57pg/ml | 18.84pg/ml |

Referring for the above Table 1, it can be confirmed that the mean value of TRAIL concentration in the cerebrospinal fluid of the control group was 2.04 pg/ml and lower than the mean value of the autoimmune encephalitis confirmed group (5.08 pg/ml) and the mean value of the viral encephalitis confirmed group (49.42 pg/ml). At this time, in case of the control group, the standard deviation was 1.99 pg/ml, the standard error of the mean was 0.63 pg/ml. And, in case of the autoimmune encephalitis confirmed group, the standard deviation was 3.08 pg/ml, the standard error of the mean was 0.97 pg/ml, and in case of the viral encephalitis confirmed group, the standard deviation was 59.57 pg/ml, the standard error of the mean was 18.84 pg/ml.

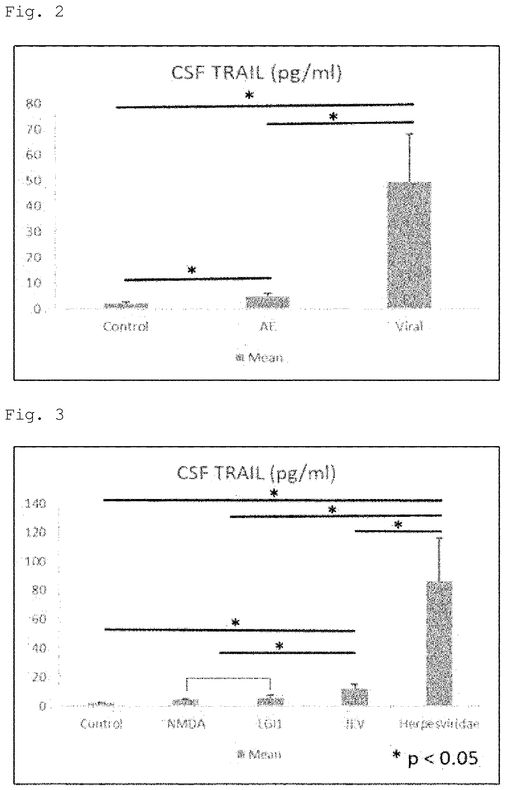

Figure 2 is a bar graph of the mean values of TRAIL concentrations in the cerebrospinal fluid of the control and experimental groups shown in Table 1. The horizontal axis of the bar graph represents the control subjects group (Control), the patient group with confirmed autoimmune encephalitis (AE), and the patient group with confirmed viral encephalitis (Viral), and the vertical axis represents concentration (pg/ml) (* means p <0.05).

From above Table 1 and figure 2, it can be seen that the mean value of TRAIL concentration in cerebrospinal fluid increases in the order of the control group, the autoimmune encephalitis confirmed group, and the viral encephalitis confirmed group. From this, it can be confirmed that patients with encephalitis and those without encephalitis can be distinguished through TRAIL concentration in cerebrospinal fluid. Furthermore, patients with autoimmune encephalitis and those with viral encephalitis can also be identified.

### 3. Analysis of TRAIL concentrations mean in cerebrospinal fluid between subdivided groups.

The below Table 2 describes Mean, Standard Deviation (SD) and Standard Error of Mean (SEM) of TRAIL concentrations in the cerebrospinal fluid of control subject groups, patient groups with anti-NMDA receptor encephalitis (NMDA), patient groups with LGI1 encephalitis (LGI1), patient groups with Japanese encephalitis virus encephalitis (JEV), and patient groups with Herpesviridae encephalitis (Herpesviridae).

**Table 2**

| Group | Mean | Standard Deviation (SD) | Standard Error of Mean (SEM) |
|---|---|---|---|
| control subjects group (Control) | 2.04pg/ml | 1.99pg/ml | 0.63pg/ml |
| patient groups with anti-NMDA receptor (NMDA) | 4.62pg/ml | 0.97pg/ml | 0.43pg/ml |
| patient groups with LGI1 (LGI1) | 5.55pg/ml | 4.46pg/ml | 1.99pg/ml |
| patient groups with JEV (JEV) | 12.14pg/ml | 6.98pg/ml | 3.12pg/ml |
| patient groups with Herpesviridae(Herpesviridae) | 86.70pg/ml | 66.79pg/ml | 29.87pg/ml |

Referring for the above Table 2, it can be confirmed that the mean value of TRAIL concentration in the cerebrospinal fluid was 2.04 pg/ml in the control group, 4.62 pg/ml in the NMDA receptor encephalitis patient group, 5.55 pg/ml in the LGI1 encephalitis patient group, 12.14 pg/ml in the Japanese Encephalitis virus encephalitis patient group, 86.70 pg/ml in Herpesviridae encephalitis patient group, indicating that the mean value of TRAIL concentration in cerebrospinal fluid was different according to the cause of encephalitis. At this time, it can be also confirmed that the standard deviation and the standard error of the mean were 1.99 pg/ml and 0.63 pg/ml in the control group, 0.97 pg/ml and 0.43 pg/ml in the anti-NMDA receptor encephalitis patient group, 4.46 pg/ml and 1.99 pg/ml in the LGI1 encephalitis patient group, 6.98 pg/ml and 3.12 pg/ml in the Japanese Encephalitis virus encephalitis patient group, 66.79 pg/ml and 29.87 pg/ml in Herpesviridae encephalitis patient group, respectively.

Figure 3 is a bar graph of the mean values of TRAIL concentrations in the cerebrospinal fluid of the control and experimental groups shown in Table 2. The horizontal axis of the bar graph represents the control subjects group (Control), anti-NMDA receptor encephalitis patient group (NMDA), LGI1 encephalitis patient group (LGI1), Japanese Encephalitis virus encephalitis patient group (JEV) and Herpesviridae encephalitis patient group (Herpesviridae), and the vertical axis represents concentration (pg/ml) (* means p <0.05).

From above Table 2 and figure 3, it can be seen that the mean value of TRAIL concentration in cerebrospinal fluid increases in the order of the control group, anti-NMDA receptor encephalitis patient group, LGI1 encephalitis patient group, Japanese Encephalitis virus encephalitis patient group and Herpesviridae encephalitis patient group. From this, it can be confirmed that patients with encephalitis and those without encephalitis can be distinguished through TRAIL concentration in cerebrospinal fluid. Furthermore, patients with autoimmune encephalitis and those with viral encephalitis can also be identified.

### 4. Receiver Operating Characteristic (ROC) analysis.

ROC Curve (Receiver Operating Characteristic Curve) means recipient manipulation characteristic, responder action characteristic, or recipient response characteristic. Sensitivity and specificity are used for ROC curve analysis. Sensitivity is a measure of how many percent of people diagnosed with the disease in the test are susceptible to disease, and specificity is a measure of how many percent of people who have not been affected are susceptible to disease.

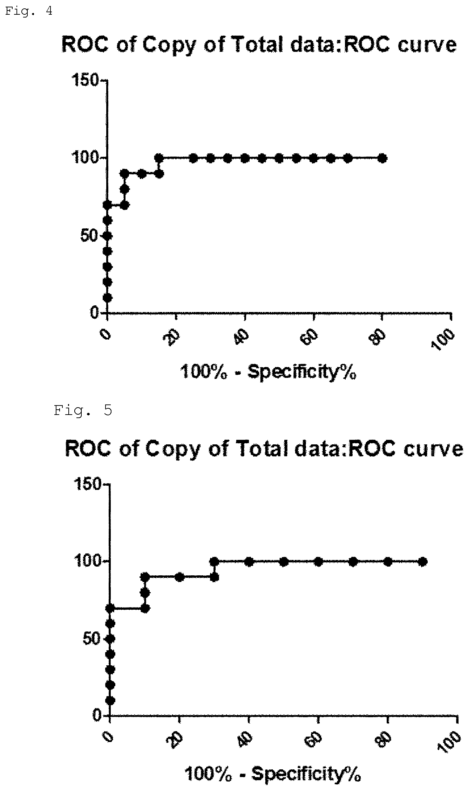

Figures 4 and 5 are graphs showing ROC curve for all 30 patients included in the control group and the experimental group, wherein the horizontal axis represents 100% - specificity (%) and the vertical axis represents sensitivity. At this time, the value of AUC is defined to be better as the discriminating ability when being closer to 1.

The ROC curve of figure 4 includes 10 control patients without encephalitis, 10 autoimmune encephalitis patients, and 10 viral encephalitis patients and shows the results when the cutoff value (reference value) is set for a case where the TRAIL concentration in cerebrospinal fluid is greater than 8.128 pg/ml.

As a result, the sensitivity and the specificity are 90% and 95%, respectively, so that viral encephalitis can be distinguished, and the AUC (Area Under the ROC Curve) value is 0.975.

The ROC curve of figure 5 includes 10 autoimmune encephalitis confirmed patient group (5 patients with anti-NMDA receptor encephalitis, 5 patients with LGI1 encephalitis) and 10 viral encephalitis confirmed patient group (5 patients with Japanese Encephalitis virus encephalitis, 5 patients with Herpesviridae encephalitis) and shows the results when the cutoff value (reference value) is set for a case where the TRAIL concentration in cerebrospinal fluid is greater than 8.128 pg/ml.

As a result, the sensitivity and the specificity are 90% and 90%, respectively, so that viral encephalitis can be distinguished, and the AUC (Area Under the ROC Curve) value is 0.950.

Referring for figures 4 and 5, it can be confirmed that the TRAIL concentration in the cerebrospinal fluid at the early stage of the symptom (before the start of treatment) can be discriminated between the normal human and the autoimmune or viral encephalitis patient with higher accuracy.

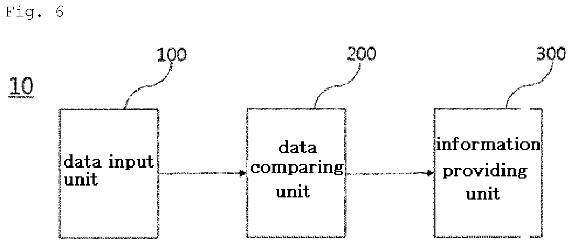

Figure 6 shows an apparatus capable of providing information related to encephalitis disorder according to an embodiment of the present invention. In the following, description of the parts described above may be omitted.

Referring for figure 6, the information providing apparatus 10 according to the present invention may include a data input unit 100, a data comparison unit 200, and an information providing unit 300.

In this case, the information providing apparatus related to encephalitis disorder may further include an extracting unit for extracting the ligand in the cerebrospinal fluid from the biological sample, and may also further include a concentration measuring unit for measuring the concentration of the ligand in the extracted ligand in the cerebrospinal fluid.

The data input unit 100 can input the concentration of the ligand in the cerebrospinal fluid obtained from the biological sample. The concentration data of the ligand in the cerebrospinal fluid to be inputted may be one that was measured from the concentration measuring unit. As described above, the concentration data may include TRAIL concentration information in cerebrospinal fluid based on TRAIL enzyme immunoassay (ELISA).

The data comparing unit 200 can compare the concentration of the ligand in the cerebrospinal fluid with the reference value. At this time, the reference value may correspond to the concentration value of the ligand in cerebrospinal fluid which is capable of discriminating the information related to the autoimmune encephalitis disorder or the viral encephalitis disease.

In particular, the data comparison unit 200 can set a reference value that increases in order of the anti-NMDA receptor encephalitis patient group, the LGI1 encephalitis patient group, the Japanese Encephalitis virus encephalitis patient group, and the Herpesviridae encephalitis patient group according to the mean value of TRAIL concentration in cerebrospinal fluid from the above Table 2 and figure 3. Thus, it is possible to discriminate patients who are not infected with encephalitis and patients with encephalitis via TRAIL concentration in cerebrospinal fluid, and to distinguish between patients with autoimmune encephalitis and patients with viral encephalitis.

For example, the data comparison unit 200 may set a cutoff (reference value) when the TRAIL concentration in cerebrospinal fluid is greater than 8.128 pg/ml to determine whether it is an autoimmune encephalitis (for example, anti-NMDA receptor encephalitis or LGI1 encephalitis) or a viral encephalitis (for example, Japanese Encephalitis virus encephalitis or Herpesviridae encephalitis).

The information providing unit 300 may output and provide to a user the information related to the autoimmune encephalitis disorder or the viral encephalitis disorder according to the data obtained by the data comparison unit 200. For example, information related to an autoimmune encephalitis disorder or information related to a viral encephalitis disorder may include the above discrimination result.

At this time, the information providing unit 300 may further include a display unit, and the said display unit may include various types of screen output means such as a liquid crystal display, a thin layer transistor liquid crystal display, an organic light emitting diode, a flexible display, a three-dimensional display.

As such, it will be understood that the technical scope of the present invention can be embodied in other specific forms without departing from the spirit or essential characteristics of the present invention.

Although the present invention has been particularly shown and described with reference to exemplary embodiments thereof for illustrative purposes, it is clearly understood that the same is by way of illustration and example only and is not to be construed to the preferred embodiments of the present invention, and that various modifications, additions and substitutions are possible, without departing from the scope and spirit of the invention as disclosed in the accompanied claims.

## Claims

1. An information providing method of an information providing apparatus comprising the steps of:
acquiring a biological sample separated from a specimen;
identifying concentration of a protein, TNF-related apoptosis-inducing ligand (TRAIL) in the cerebrospinal fluid from the said biological sample;
comparing the concentration of the said identified TRAIL to a reference value; and
providing information related to encephalitis disorder according to the said results of comparison.

2. The information providing method of claim 1, wherein the said comparing step further comprise the steps of:
discriminating the possibility of an autoimmune encephalitis disorder if the concentration of the identified TRAIL is less than a reference value according to the result of the comparison; and
discriminating the possibility of a viral encephalitis disorder if the concentration of the identified TRAIL is greater than a reference value according to the result of the comparison.

3. The information providing method of claim 2, wherein the said information providing method further comprise the steps of:
providing viral encephalitis diagnostic information according to the viral encephalitis disorder possibility discrimination; and
providing autoimmune encephalitis diagnostic information according to the autoimmune encephalitis disorder possibility discrimination.

4. The information providing method of claim 2, wherein the said step of discriminating the possibility of autoimmune encephalitis disorder comprises a step of discriminating between anti-NMDA receptor encephalitis and LGI1 encephalitis according to the said identified TRAIL concentration.

5. The information providing method of claim 2, further comprising a step of discriminating with normal without encephalitis when the said concentration of the TRAIL is lower than a predetermined value.

6. The information providing method of claim 2, wherein the said step of discriminating the possibility of the viral encephalitis disorder comprises a step of discriminating Japanese Encephalitis virus (JEV) encephalitis and Herpesviridae encephalitis according to the said identified TRAIL concentration.

7. The information providing method of claim 1, wherein it is be characterized that the said concentration of TRAIL in the cerebrospinal fluid is obtained from an enzyme immunoassay (ELISA).

8. An apparatus for providing information comprising:
a data input unit receiving data including a concentration of TRAIL (TNF-related apoptosis-inducing ligand) which is a protein in cerebrospinal fluid obtained from a biological sample;
a data comparing unit comparing the said concentration of TRAIL with the reference value; and
an information providing unit providing information related to encephalitis disorder according to the said comparison result.

9. The apparatus for providing information of claim 8, wherein the said information providing unit provides information related to an autoimmune encephalitis disorder when the concentration of the TRAIL is lower than a reference value according to the said results of comparison, and provides information related to a viral encephalitis disorder when the concentration of the TRAIL is greater than a reference value according to the said results of comparison.

10. The apparatus for providing information of claim 9, wherein the said information related to a viral encephalitis disorder includes the diagnostic information according to discrimination of the possibility of a viral encephalitis disorder, and the said information related to an autoimmune encephalitis disorder includes the diagnostic information according to discrimination of the possibility of a autoimmune encephalitis disorder.

11. The apparatus for providing information of claim 8, wherein the said information providing unit distinguishes between the anti-NMDA receptor encephalitis and the LGI1 encephalitis according to the said results of comparison.

12. The apparatus for providing information of claim 8, wherein the said information providing unit distinguishes JEV (Japanese Encephalitis virus) encephalitis and Herpesviridae encephalitis according to the said results of comparison.
